# EUROPEAN PATENT APPLICATION

(11) **EP 2 489 310 A1**
(43) Date of publication of application: **22.08.2012**
(21) Application number: 12000808.1
(22) Date of filing: 08.02.2012
(51) Int. Cl.: A61B 6/03

(54) **Rotation sensor for use with an imaging system and method for using the same**

(30) Priority: 16.02.2011 US 201113028391
(71) Applicant: Morpho Detection, Inc., Newark, CA 94560 (US)
(72) Inventor: Tang, David, Newwark CA 94560 (US); Ingermann, Eugene Alex, San Francisco CA 94112 (US)
(74) Representative: Müller-Boré & Partner Patentanwälte

(57) **Abstract**

A control system (18) for use with an imaging system (10) is provided. The control system includes a rotation sensor (12) coupled to a rotating portion (20) of a gantry (14) of the imaging system. The rotation sensor includes a gyroscope (30) configured to measure (106) a rotation parameter of the gantry. The control system further includes a data acquisition system (DAS) (78) coupled in communication with the rotation sensor and a detector (26) of the imaging system. The DAS is configured to associate (120) projection data from the detector with the measured rotation parameter.

## Description

### BACKGROUND OF THE INVENTION

The embodiments described herein relate generally to a rotation sensor for use with an imaging system and, more particularly, to a rotation sensor that includes a gyroscope for measuring a rotation parameter of an imaging system.

At least some known imaging systems include a gantry having a portion that rotates about an object to be imaged. In such imaging systems, gantry angular position information is used to reconstruct images. As such, at least some known imaging systems include magnetic and/or optical encoders to sense gantry position. However, such encoders typically have relatively high initial and replacement costs. Further, such encoders are susceptible to contamination by dust, metal particulates, and/or other debris. Moreover, encoder bands include many fragile small pulses that are susceptible to being damaged by contaminants and/or magnetic objects. When these pulses are damaged, replacing the encoder band on a fielded unit can be costly in terms of monetary cost and machine downtime.

At least one other imaging system uses accelerometers to correct for beam misalignment. Another known imaging system uses laser gyros to separately measure positions of a cathode and positions of an anode. However, such an imaging system does not measure rotation of a gantry.

### BRIEF SUMMARY OF THE INVENTION

In one aspect, a control system for use with an imaging system is provided. The control system includes a rotation sensor coupled to a rotating portion of a gantry of the imaging system. The rotation sensor includes a gyroscope configured to measure a rotation parameter of the gantry. The control system further includes a data acquisition system (DAS) coupled in communication with the rotation sensor and a detector of the imaging system. The DAS is configured to associate projection data from the detector with the measured rotation parameter.

In another aspect, an imaging system is provided. The imaging system includes a gantry having a stationary portion and a rotating portion that rotates with respect to the stationary portion and a radiation detector coupled to the gantry and configured to rotate with the gantry. The radiation detector is configured to detect radiation emitted from a radiation source to acquire projection data. The imaging system further includes a control system having a rotation sensor coupled to the rotating portion. The rotation sensor includes a gyroscope configured to measure a rotation parameter of the gantry. The control system is configured to associate the projection data from the radiation detector with the measured rotation parameter.

In yet another aspect, a method for generating an image using an imaging system is provided. The imaging system includes a gantry having a rotating portion and a stationary portion. The rotating portion includes a radiation source, a radiation detector, and a rotation sensor. The method includes rotating the rotating portion of the gantry with respect to the stationary portion of the gantry, measuring a rotation parameter of the gantry using the rotation sensor that includes a gyroscope, and collecting projection data from the radiation detector. The projection data is associated with the measured rotation parameter of the gantry.

### BRIEF DESCRIPTION OF THE DRAWINGS

Figs. 1-5 show exemplary embodiments of the system and method described herein.

Fig. 1 is a perspective view of an exemplary multi-slice computed tomography (CT) system.

Fig. 2 is a block diagram of the CT system shown in Fig. 1.

Fig. 3 is a schematic diagram of an exemplary embodiment of a rotation sensor that may be used with the CT system shown in Figs. 1 and 2.

Fig. 4 is a schematic diagram of an alternative embodiment of a rotation sensor that may be used with the CT system shown in Figs. 1 and 2.

Fig. 5 is a flowchart illustrating an exemplary method that may be used with the CT system shown in Figs. 1-4.

### DETAILED DESCRIPTION OF THE INVENTION

The embodiments described herein include a rotation sensor, such as a gyroscope, that can be used to measure an angular position and/or other rotation parameter of a gantry of an imaging system. In a particular embodiment, one or more gyroscopes are mounted onto the gantry, a data acquisition system is interfaced to the gyroscope(s) to get an angular rate measurement, and a processor integrates the angular rate measurements over time to calculate an angular position of the gantry. Alternatively, the gyroscope(s) can be configured to output angular position signals. High precision angular position calculations can be made after a combination of calibrating the gyroscope(s) after being mounted onto the gantry and calibrating the gyroscope during rotation by using gantry position indicator(s), such as magnets. These gantry position indicator(s) give high-accuracy gantry position information, which is used to calibrate the gyroscope(s). The rotation sensor can provide raw or conditioned signals to the data acquisition system to trigger projection data collection and/or correct collected projection data.

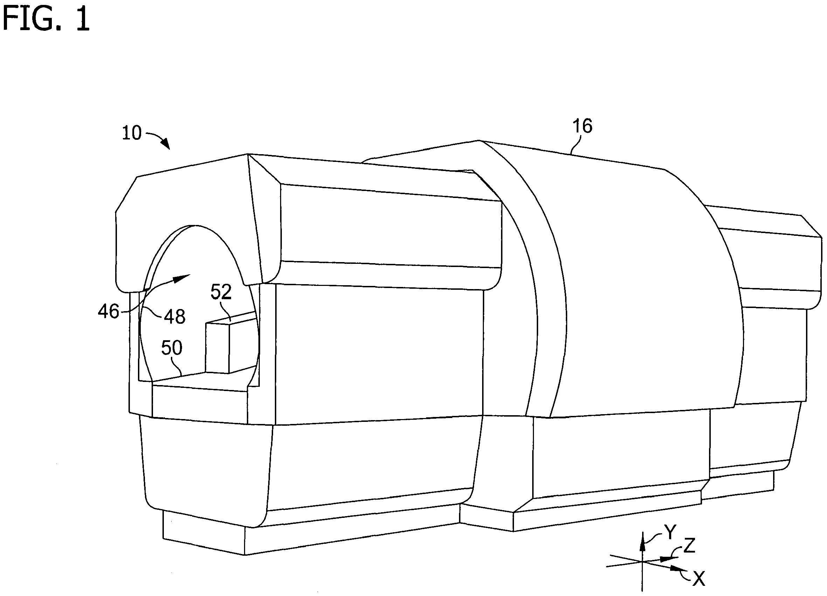

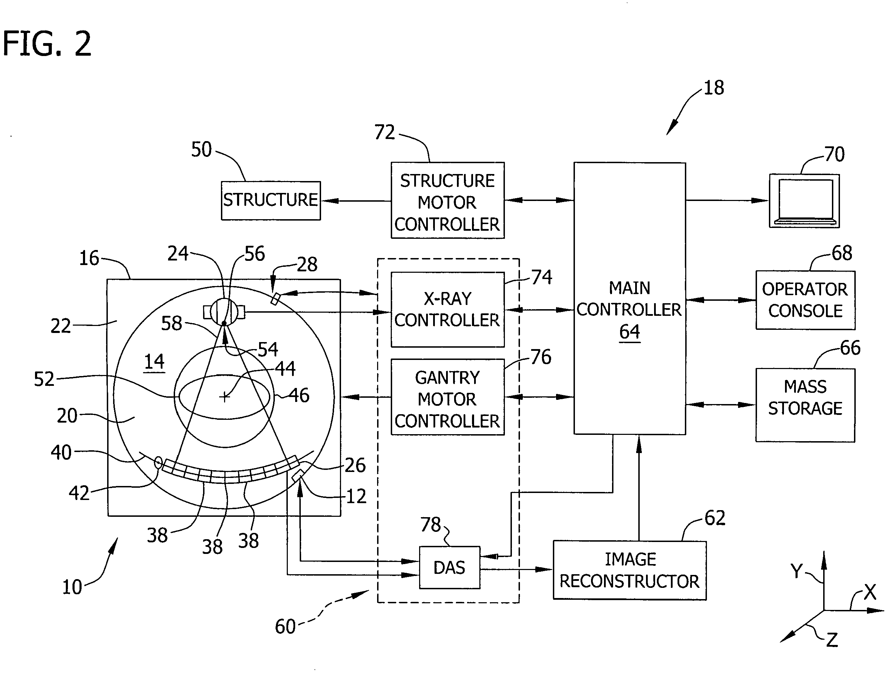

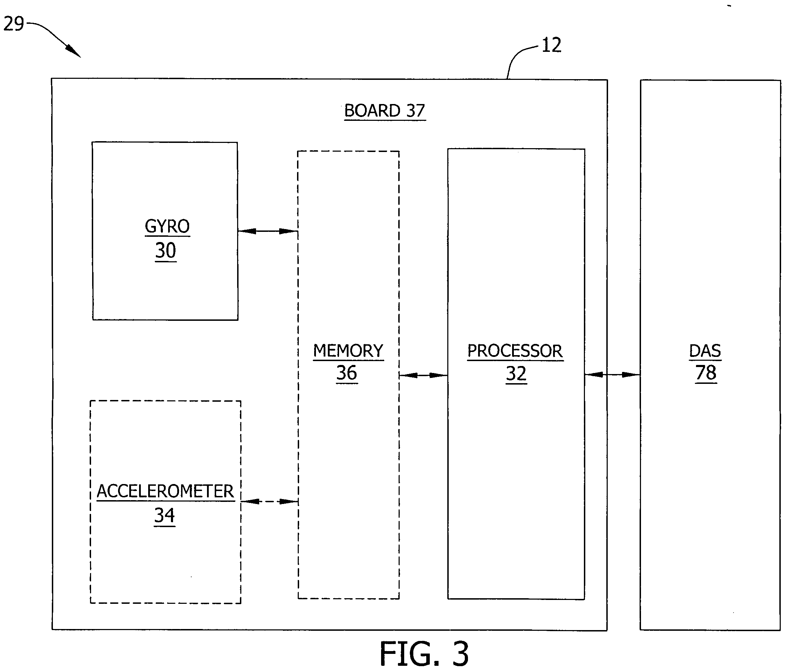

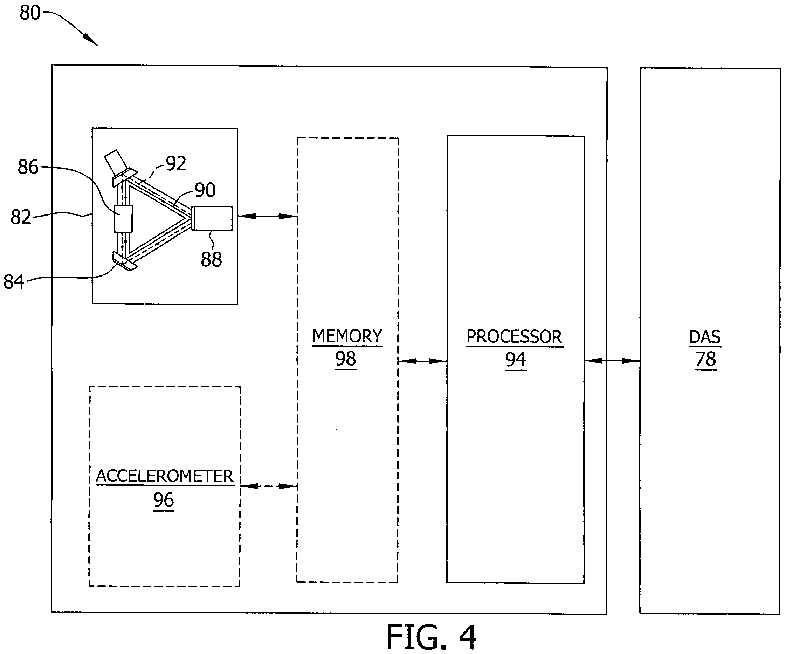

Although a computed tomography (CT) system is described herein, it should be understood that the embodiments described herein can be used with any suitable imaging system having at least one rotating component. Fig. 1 is a perspective view of an exemplary multi-slice CT system 10. Fig. 2 is a block diagram of CT system 10. Fig. 3 is a schematic diagram of an exemplary embodiment of rotation sensor 12 that may be used with CT system 10. Fig. 4 is a schematic diagram of an alternative embodiment of rotation sensor 12 that may be used with CT system 10.

Referring to Figs. 1 and 2, an exemplary embodiment of CT system 10 includes multiple operational modes to yield high throughputs. In one embodiment, CT system 10 is integrated into an airport installation; however, CT system 10 may be integrated into any suitable installation, such as a non-destructive testing installation and/or a medical installation. In the exemplary embodiment, CT system 10 includes a gantry 14 within a housing 16. A control system 18 controls operation of CT system 10. Gantry 14 includes an inner rotating portion 20 and a stationary portion 22. Rotating portion 20 is configured to rotate with respect to stationary portion 22. Rotating portion 20 includes a radiation source, such as an X-ray source 24, a radiation detector, such as a detector array 26, and at least one rotation sensor 12 coupled thereto. At least one gantry position indicator 28 is coupled to rotating portion 20 and/or stationary portion 22. Control system 18 includes rotation sensor 12 and position indicator 28. More than one rotation sensor 12 and/or more than one position indicator 28 can be coupled to gantry 14, however, for the sake of clarity, one rotation sensor 12 and one position indicator 28 are described herein.

Rotation sensor 12 is coupled to a controller board within control system 18 using a cable that provides at least power to rotation sensor 12 and signal communication with rotation sensor 12. Alternatively, rotation sensor 12 is mounted on a controller board configured to control detector array 26. As such, electrical connections between rotation sensor 12 and the remainder of the controller board are integrated into the controller board.

Referring to Fig. 3, in one embodiment, rotation sensor 12 is a microelectromechanical system (MEMS) 29 that includes at least a gyroscope 30. In a particular embodiment, MEMS 29 includes a three-axis gyroscope and a three-axis accelerometer. MEMS 29 can further include a processor 32, an accelerometer 34, and/or a memory 36. In the exemplary embodiment, MEMS 29 includes gyroscope 30 and processor 32 on a shared board 37. In an alternative embodiment, MEMS 29 includes gyroscope 30, and processor 32 is separate from gyroscope 30, such as positioned at any suitable location within control system 18.

Referring to Fig. 4, in an alternative embodiment, rotation sensor 12 is a ring laser system 80 that includes a laser gyroscope 82. More specifically, ring laser system 80 includes any suitable laser gyroscope, such as a passive laser gyroscope or an active laser gyroscope. In the exemplary embodiment, laser gyroscope 82 includes at least one mirror 84, a laser source 86, and a readout sensor 88. Laser source 86 is configured to produce a clockwise laser beam 90 and a counter-clockwise laser beam 92. Readout sensor 88 is configured to translate a moving interference pattern of laser beams 90 and 92 into digital pulses, wherein each pulse represents an angle of rotation. Ring laser system 80 can further include a processor 94, an accelerometer 96, and/or a memory 98. In the exemplary embodiment, ring laser system 80 includes gyroscope 82 and processor 94. In an alternative embodiment, ring laser system 80 includes gyroscope 82, and processor 94 is separate from gyroscope 82, such as positioned at any suitable location within control system 18.

Alternatively, rotation sensor 12 is any suitable sensor that enables CT system 10 to function as described herein and is not limiting to being MEMS 29 and/or ring laser system 80. Referring to Figs. 1-4, in the exemplary embodiment, rotation sensor 12 is configured to measure at least one rotation parameter of gantry 14. Rotation parameters include, but are not limited to including, an angular position, an angular velocity, an angular acceleration, and/or a change in the angular acceleration (i.e. angular jerk) of rotating portion 20 with respect to stationary portion 22. Rotation sensor 12 measures and/or senses at least one rotation parameter of gantry rotating portion 20 using a gyroscope, such as gyroscope 30 or 82, and/or any other suitable sensor, such as accelerometer 34. Further, processor 32 is configured to correct a signal generated by at least the gyroscope for use by CT system 10, as described in more detail below. Rotation sensor 12 can periodically and/or continuously output the signal for use in CT system 10.

Referring to Figs. 1 and 2, in the exemplary embodiment, gantry position indicator 28 is any suitable indicator that indicates a position of rotating portion 20 with respect to stationary portion 22. In one embodiment, gantry position indicator 28 includes a magnetic position indicator. Gantry position indicator 28 is coupled in communication with control system 18, which is configured to use signals from gantry position indicator 28 to calibrate rotation sensor 12, as described in more detail below.

Detector array 26 includes multiple rows of detector elements 38 and/or multiple channels of detector elements 38. The detector channels are parallel to a channel axis 40, which is parallel to a plane of gantry 14. The detector rows are parallel to a row axis 42, which is parallel to a Z-axis. Each detector row is displaced from all other detector rows in a Z-direction along a center axis 44 about which gantry 14 rotates. Center axis 44 is substantially parallel to the Z-axis. Alternatively, any suitable detector and/or detector array is used within CT system 10. In the exemplary embodiment, an examination zone 46 is defined within housing 16 between X-ray source 24 and detector array 26. Examination zone 46 is accessed through an opening 48 in housing 16.

CT system 10 further includes a support structure 50 within examination zone 46. Support structure 50 is configured to translate an object 52 along center axis 44, parallel to the Z-direction, between X-ray source 24 and detector array 26 to perform a helical scan of examination zone 46, or is configured to maintain the position of object 52 along center axis 44 throughout an axial scan of object 52. In one embodiment, support structure 50 translates object 52 in a Y-direction and/or in an X-direction. In the exemplary embodiment, support structure 50 is a conveyor apparatus. During operation, support structure 50 conveys object 52 into examination zone 46. X-ray source 24 and detector array 26 revolve with rotation of gantry rotating portion 20. More specifically, X-ray source 24 and detector array 26 rotate about center axis 44 such that X-ray source 24 and detector array 26 rotate about object 52 placed on support structure 50.

X-ray source 24 includes a focal spot 54 having a center 56. X-ray source 24 generates a beam 58 of X-rays and projects beam 58 towards detector array 26. Beam 58, after passing through object 52, is detected at detector array 26 to generate projection data that is used to create a CT image (not shown) of object 52. As beam 58 passes through object 52, beam 58 is attenuated and may create scattered radiation (not shown). In one embodiment, scattered radiation at a predetermined angle (not shown) to beam 58 passes through a secondary collimator (not shown) and is detected by a scatter detector (not shown) and/or by multiple detector elements 38 in multiple detector rows of detector array 26. In the exemplary embodiment, detector elements 38 generate projection data, which represent electrical signals corresponding to intensities of beam 58.

CT system 10 includes control system 18 having a plurality of components to enable operation of CT system 10. In the exemplary embodiment, control system 18 includes rotation sensor 12, a control mechanism 60, an image reconstructor 62, a main controller 64, a mass storage device 66, an operator console 68, a display monitor 70, and a support structure motor controller 72. In the exemplary embodiment, rotation of gantry 14, an operation of X-ray source 24, and an operation of detector array 26 are governed by control mechanism 60. Control mechanism 60 includes an X-ray controller 74 that provides power and timing signals to X-ray source 24 and a gantry motor controller 76 that controls a speed and/or rotation and a position of gantry rotating portion 20.

A data acquisition system (DAS) 78 samples projection data from detector elements 38. More specifically, rotation sensor 12 is coupled in communication with DAS 78 and transmits signals representing the measured rotation parameter of gantry 14 to DAS 78. In the exemplary embodiment, rotation sensor 12 transmits the rotation parameter, such as the angular position, the angular velocity, the angular acceleration, and/or the change in the angular acceleration of gantry rotating portion 20, to DAS 78. DAS 78 is configured to associate the measured rotation parameter with the projection data. In one embodiment, DAS 78 associates the data sets by controlling acquisition of data from detector array 26 based on the signals from rotation sensor 12. For example, DAS 78 triggers data collection from detector array 26 based on the measured rotation parameter. In an alternative embodiment, DAS 78 associates the data sets by correcting the projection data using the measured rotation parameter as the projection data is acquired or after the projection data and the measured rotation parameters have been acquired. In the exemplary embodiment, DAS 78 converts the projection data from an analog form to digital signals to generate sampled and digitized projection data, which is actual projection data. Image reconstructor 62 receives the actual projection data from DAS 78 and performs image reconstruction to generate the CT image.

Main controller 64 stores the projection data, the measured rotation parameters, and/or the CT image in mass storage device 66. Examples of mass storage device 66 include a nonvolatile memory, such as a read only memory (ROM), and a volatile memory, such as a random access memory (RAM). Other examples of mass storage device 66 include a floppy disk, a compact disc-ROM (CD-ROM), a magneto-optical disk (MOD), and a digital versatile disc (DVD). Further, main controller 64 also receives commands and scanning parameters from an operator (not shown) via operator console 68. Display monitor 70 allows the operator to observe the CT image and other data from main controller 64. The operator supplied commands and parameters are used by main controller 64 in operation of DAS 78, X-ray controller 74, and/or gantry motor controller 76. In addition, main controller 64 operates support structure motor controller 72, which translates support structure 50 to position object 52 within system housing 16. Moreover, in the exemplary embodiment, main controller 64 uses computer algorithms to analyze the image and compare CT properties of the image with CT properties of known contraband materials. If a match is found, main controller 64 sounds an alarm and/or displays an image of object 52 on display monitor 70 such that the operator may view the image to determine whether a real threat exists.

Processor 32, X-ray controller 74, gantry motor controller 76, DAS 78, image reconstructor 62, main controller 64, and/or structure motor controller 72 are not limited to only those integrated circuits referred to in the art as a controller, but broadly refers to a computer, a processor, a microcontroller, a microcomputer, a programmable logic controller, an application specific integrated circuit, firmware, a circuit, software, and/or any other programmable circuit. Processor 32, X-ray controller 74, gantry motor controller 76, DAS 78, image reconstructor 62, main controller 64, and/or structure motor controller 72 may be a portion of a central control unit (not shown) or may each be a stand-alone component, as shown. Further, although the embodiment described above refers to a third generation CT imaging system, rotation sensor 12, as described herein, may be coupled to fourth generation CT systems that have a stationary detector and a rotating X-ray source, industrial imaging system in which an object is rotated with respect to a detector and/or X-ray source, and/or future generations of CT systems involving at least one rotating component.

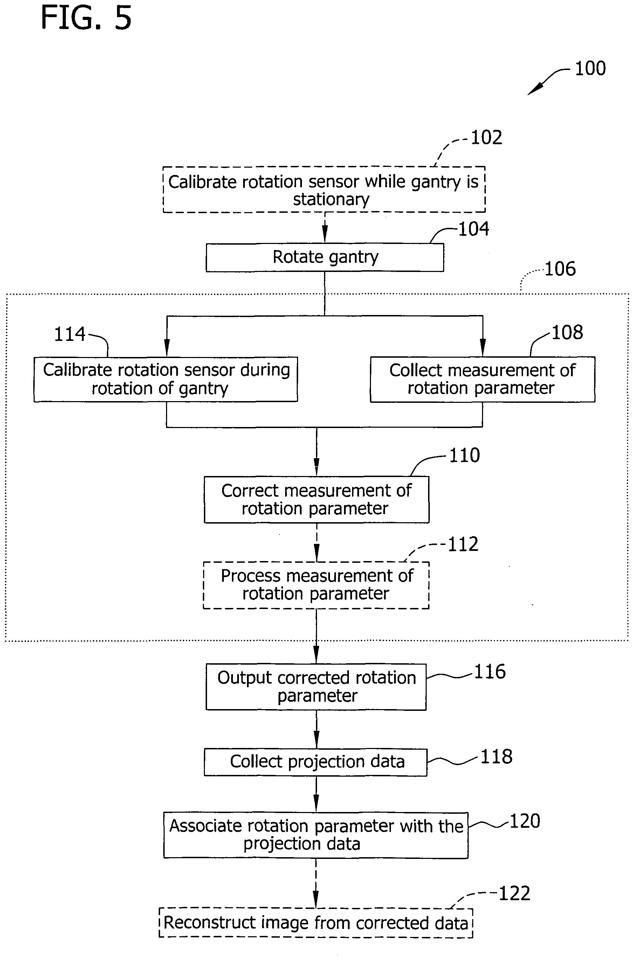

Control system 18 and/or processor 32 are configured to perform the methods described herein. Fig. 5 is a flowchart illustrating an exemplary method 100 that may be used with CT system 10 (shown in Figs. 1-4). By performing method 100, projection data representing object 52 (shown in Figs. 1 and 2) can be collected and an image of object 52 can be reconstructed. Method 100 is performed at least partially by control system 18 (shown in Fig. 2) sending or transmitting commands and/or instructions to components of CT system 10, such as rotation sensor 12 (shown in Figs. 2-4), detector array 26 (shown in Fig. 2), and/or any other suitable component. Processor 32 (shown in Fig. 3), processor 94 (shown in Fig. 4), DAS 78 (shown in Fig. 2), and/or another suitable processor and/or controller within control system 18 is programmed with code segments configured to perform method 100. Alternatively, method 100 is encoded on a computer-readable medium that is readable by control system 18. In such an embodiment, control system 18 is configured to read the computer-readable medium for performing method 100.

Method 100 enables data collection to be triggered and/or corrected more precisely than in conventional CT systems. More specifically, measurements from rotation sensor 12 enable DAS 78 to precisely acquire projection data at predetermined angular positions and/or at a predetermined angular rate and/or precisely correct projection data for the angular position at which the projection data is acquired. For example, projection data is collected from detector array 26 at an interval of degrees or fractions of degrees, and a measured rotation parameter from rotation sensor 12 is associated with the projection data as the projection data is collected. In another example, projection data is correcting using a measured rotation parameter from rotation sensor 12 while the projection data is being acquired and/or after a set of projection data has been acquired.

By associating a precise rotation parameter with the collected projection data, an image can more accurately be reconstructed, as compared with CT systems that do not include rotation sensor 12. As DAS 78 collects projection data from detector array 26, DAS 78 time-stamps the projection data, which facilitates mapping the projection data to a measured rotation parameter at the time of projection data collection. Because the collection and/or correction of projection data is more precise as compared to conventional CT systems, an image reconstructed using method 100 has better image quality and/or fewer artifacts as compared to images reconstructed using conventional CT systems and methods.

For the sake of clarity MEMS 29 is referred to below to describe method 100; however, it should be understood that ring laser system 80 can also be used to perform method 100. Referring to Figs. 1-3 and 5, method 100 includes calibrating 102 rotation sensor 12 while gantry rotating portion 20 is stationary. More specifically, rotation sensor 12 is initially calibrated 102, and the initial calibration data is stored in control system 18, such as in memory 36. Rotation sensor 12 can be calibrated 102 using any suitable method and/or technique. For example, rotation sensor 12 is calibrated 102 by using signals from gantry position indicator 28. In a particular embodiment, rotation sensor 12 is calibrated 102 by capturing a signal from gyroscope 30 when gantry rotating portion 20 is stationary and using the captured signal to calculate a bias correction and environmental effects, such as temperature. Signals from gyroscope 30 are then captured while gantry rotating portion 20 is rotating, and the signals are corrected using position information provided by position indicator 28. In an alternative embodiment, rotation sensor 12 and/or gyroscope 30 is configured to perform initial calibration 102. Alternatively, rotation sensor 12 is not initially calibrated 102 and only calibrated 114 during rotation 104 of gantry rotating portion 20.

In the exemplary embodiment, after rotation sensor 12 is initially calibrated 102, gantry rotating portion 20 is rotated 104 with respect to gantry stationary portion 22. In the exemplary embodiment, rotation 104 of rotating portion 20 is controlled by gantry motor controller 76. While rotating portion 20 is rotated 104, rotation sensor 12 measures 106 a rotation parameter of gantry 14. In the exemplary embodiment, rotation sensor 12 measures 106 an angular position, an angular velocity, an angular acceleration, and/or an angular jerk of rotating portion 20. More specifically, gyroscope 30 and/or accelerometer 34 collects 108 raw measurement data, and processor 32 and/or control system 18 corrects 110 and/or otherwise processes 112 the raw measurement data to generated the measured rotation parameter. In the exemplary embodiment, the raw measurement data is corrected 110 for systematic error and/or random error. In one embodiment, the raw measurement data is corrected 110 for sensor offset, sensitivity, linear acceleration error, random error, temperature error, and/or sensor calibration.

In the exemplary embodiment, rotation sensor 12 is calibrated 114 while rotating portion 20 is rotated 104 using, for example, position indicator 28. More specifically, rotation sensor 12 is periodically calibrated 114 during rotation 104 of rotating portion 20. For example, rotation sensor 12 is calibrated 114 once or twice per rotation of rotating portion 20. In an alternative embodiment, rotation sensor 12 is calibrated 114 at predetermined time intervals. Alternatively, rotation sensor 12 is calibrated at any suitable time, either automatically or manually upon an operator's instruction. In the exemplary embodiment, processor 32 and/or control system 18 corrects 110 the raw measurement data to account for calibration 114 of rotation sensor 12, in addition to correcting 110 for system and/or random error.

Further, processor 32 and/or control system 18 processes 112 the corrected measurement data and/or the raw measurement data to generate a predetermined rotation parameter, if rotation sensor 12 does not directly measure the predetermined rotation parameter. For example, if rotation sensor 12 measures angular velocity as the raw measurement data and an angular position is desired, processor 32 and/or control system 18 processes 112 the raw measurement data to determine the angular position from the angular velocity. Alternatively, the raw measurement data undergoes any suitable processing 112 to generate the measured rotation parameter. In a particular embodiment, the raw measurement data is corrected 110 but not processed 112.

Rotation sensor 12 outputs 116 the corrected and/or processed measurement data as the measured rotation parameter. More specifically, rotation sensor 12 outputs 116 the measured rotation parameter to DAS 78. Rotation sensor 12 outputs 116 the measured rotation parameter continuously and/or periodically in substantially real-time. Alternatively, rotation sensor 12 automatically and/or on request outputs 116 the measured rotation parameter at any suitable time.

In the exemplary embodiment, DAS 78 and/or control system 18 collects 118 projection data from detector array 26 and associates 120 the projection data with the measured rotations parameter. In one embodiment, the projection data is associated 120 with the measured rotation parameter by triggering collection 118 of the projection data based on the measured rotation parameter of gantry 14. As such, collection 118 and association 120 are performed substantially simultaneously. More specifically, as rotating portion 20 rotates 104 and rotation sensor 12 outputs 116 the measured rotation parameter, X-ray source 24 generates beam 58 and attenuated and/or scattered radiation from beam 58 is detected at detector array 26. In the exemplary embodiment, X-ray source 24 continuously generates beam 58 and radiation is continuously detected at detector array 26. However, data representing the detected radiation is collected 118 from detector array 26 by DAS 78 based on the measured rotation parameter. More specifically, in the exemplary embodiment, projection data is collected 118 at predetermined angular positions and/or at a predetermined angular rate based on the measured rotation parameter. As such, the measured rotation parameter indicates angular position and/or angular rate in such an embodiment. Alternatively, projection data is collected 118 using any suitable measurement that is indicated by the measure rotation parameter.

In an alternative embodiment, the projection data is associated 120 with the measured rotation parameter by correcting the projection data using the measured rotation parameter of gantry 14. As such, collection 118 and association 120 are performed substantially simultaneously and/or at different times. When collection 118 and association 120 are performed substantially simultaneously, a set of projection data and a set of measured rotation parameters are acquired separately and associated 120 at substantially real time. When collection 118 and association 120 are performed separately, a set of projection data and a set of measured rotation parameters are acquired separately and associated 120 subsequent to collection 118 of the projection data.

More specifically, in the alternative embodiment, as rotating portion 20 rotates 104 and rotation sensor 12 outputs 116 the measured rotation parameter, X-ray source 24 generates beam 58 and attenuated and/or scattered radiation from beam 58 is detected at detector array 26. In the exemplary embodiment, X-ray source 24 continuously generates beam 58 and radiation is continuously detected at detector array 26. Data representing the detected radiation is collected 118 substantially continuously from detector array 26 by DAS 78 to generate a set of projection data. Substantially simultaneously with collection 118, the measured rotation parameter is received substantially continuously by DAS 78 to generate a set of measured rotation parameters. The set of projection data is associated 120 with the set of measured rotation parameters as the sets are acquired or after the sets have been acquired. When the association 120 occurs after the collection 118, the set of projection data and the set of measured rotation parameters can be stored for subsequent association 120.

Further, in the alternative embodiment, to associate 120 the set of projection data with the set of measured rotation parameters, DAS 78 corrects the projection data using the measured rotation parameters during or after collection 118. More specifically, DAS 78 corrects the projection data to account for a rotational position at which the projection data was acquired. For example, detector array 26 measures cumulative X-ray radiation that passing through object 52 being scanned. A measurement of the cumulative X-ray radiation passing through object 52 depends on physical properties of object 52 and detector exposure time. Such a measurement is also referred to herein as a measured cumulative X-ray exposure. CT system 10 typically uses a nominal exposure time, which corresponds to rotating gantry angular position change, in a reconstruction algorithm. The measured cumulative X-ray exposure is corrected by scaling the measurement using measured deviation from the nominal exposure time to improve the image quality.

In the exemplary embodiment, DAS 78 further time stamps measured rotation parameters and/or projection data as the measured rotation parameters are output 116 and/or as the projection data is collected 118. As such, a time and measured rotation parameter is stored with projection data at each instance of data collection 118 from detector array 26. Alternatively, parameter time stamps are stored with the measured rotation parameters and projection time stamps are stored with the projection data such that the measured rotation parameters can be associated 120 with the projection data by matching the parameter time stamps with the projection time stamps. Further, in the exemplary embodiment, raw measurement data continues to be corrected 110 and rotation sensor 12 continues to be calibrated 114 as projection data is collected 118.

Image reconstructor 62 and/or control system 18 reconstructs 122 an image from the collected projection data as associated 120 with the measured rotation parameter. More specifically, image reconstructor 62 and/or control system 18 uses at least the measured rotation parameter associated with the projection data to reconstruct 122 the image. By using the measured rotation parameter at which the projection data was collected 118 and the time at which the projection data was collected 118, image reconstructor 62 and/or control system 18 reconstructs 122 an image having fewer artifacts and/or higher quality than images reconstructed using conventional CT systems.

The above-described embodiments provide a rotation sensor for triggering the collection of image data. More specifically, the rotation sensor described herein includes a gyroscope within a microelectromechanical system (MEMS) and/or a ring laser system. The gyroscope provides precise angle measurements to a data acquisition system for collecting and/or correcting image data. As such, the rotation sensor improves image quality as compared to known imaging systems that do not include such a gyroscope. Further, because the rotation sensor is a MEMS or a ring laser system, the rotation sensor lowers a cost of the imaging system and servicing costs of the imaging system. Further, the MEMS or a ring laser system is less susceptible to contamination by debris and are robust and, as such, the imaging system described herein has improved reliability as compared to imaging systems that have magnetic and/or optical encoders. Moreover, the rotation sensor and/or gyroscope is mounted onto a gantry rotating frame and interfaces with detector array with as few as one cable. Additionally, the imaging system described herein is relatively easy to assemble during manufacturing and to replace in the field.

A technical effect of the systems and method described herein includes at least one of: (a) rotating a rotating portion of a gantry with respect to a stationary portion of the gantry; (b) measuring a rotation parameter of the gantry using a rotation sensor that includes a gyroscope; (c) associating the measured rotation parameter with collected projection data; (d) collecting projection data from a radiation detector based on the measured rotation parameter of the gantry; (e) collecting the projection data at predetermined angular positions that are indicated by the measured rotation parameter; (f) collecting the projection data at a predetermined angular rate that is indicated by the measured rotation parameter; and (g) correcting the projection data using the measured rotation parameter.

Exemplary embodiments of a rotation sensor for use with an imaging system and method for using the same are described above in detail. The methods and systems are not limited to the specific embodiments described herein, but rather, components of systems and/or steps of the methods may be utilized independently and separately from other components and/or steps described herein. For example, the methods may also be used in combination with other imaging systems and methods, and are not limited to practice with only the CT systems and methods as described herein. Rather, the exemplary embodiment can be implemented and utilized in connection with many other imaging applications.

Although specific features of various embodiments of the invention may be shown in some drawings and not in others, this is for convenience only. In accordance with the principles of the invention, any feature of a drawing may be referenced and/or claimed in combination with any feature of any other drawing.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A control system (18) for use with an imaging system (10), said control system comprising:
a rotation sensor (12) coupled to a rotating portion (20) of a gantry (14) of the imaging system, said rotation sensor comprising a gyroscope (30) configured to measure (106) a rotation parameter of the gantry; and
a data acquisition system (DAS) (78) coupled in communication with said rotation sensor and a detector (26) of the imaging system, said DAS configured to associate (120) projection data from the detector with the measured rotation parameter.

2. A control system (18) in accordance with Claim 1, wherein said rotation sensor (12) is configured to measure (106) at least one of angular position, angular velocity, angular acceleration, and angular jerk.

3. A control system (18) in accordance with Claim 1 or 2, wherein said DAS (78) is configured to:
collect (108, 118) a set of measured rotation parameters and a set of projection data; and
correct (110, 112) the set of projection data using the set of measured rotation parameters.

4. A control system (18) in accordance with any of Claims 1 to 3, wherein said DAS (78) is configured to collect (118) data from said detector (26) at predetermined angular positions that are indicated by the measured rotation parameter.

5. A control system (18) in accordance with any of Claims 1 to 4, wherein said rotation sensor (12) comprises one of a microelectromechanical system (MEMS) (29) and a ring laser system (80).

6. A control system (18) in accordance with Claim 5, wherein said ring laser system (80) comprises a laser gyroscope (82).

7. A control system (18) in accordance with Claim 5, wherein said MEMS (29) comprises a gyroscope (30).

8. A control system (18) in accordance with any of Claims 5 to 7, wherein said one of said MEMS (29) and said ring laser system (80) further comprises at least one of an accelerometer (34, 96), a processor (32, 94), and a memory (36,98).

9. A method (100) for generating an image using an imaging system (10) including a gantry (14) having a rotating portion (20) and a stationary portion (22), the rotating portion including a radiation source (24), a radiation detector (26), and a rotation sensor (12), said method comprising:
rotating (104) the rotating portion of the gantry with respect to the stationary portion of the gantry;
measuring (106) a rotation parameter of the gantry using the rotation sensor, the rotation sensor including a gyroscope (30);
collecting (118) projection data from the radiation detector; and
associating (120) the projection data with the measured rotation parameter of the gantry.

10. A method (100) in accordance with Claim 9, wherein measuring (106) a rotation parameter of the gantry (14) comprises:
collecting (108) raw measurement data using the gyroscope (30); and
correcting (110, 112) the raw measurement data to generate the rotation parameter.

11. A method (100) in accordance with Claim 10, wherein correcting (110, 112) the raw measurement data comprises correcting (110) the raw measurement data for at least one of sensor offset, sensitivity, linear acceleration error, random error, and temperature error.

12. A method (100) in accordance with any of Claims 9 to 11 further comprising calibrating (102, 114) the rotation sensor (12) when at least one of the rotating portion (20) of the gantry (14) is rotating and the rotating portion of the gantry is stationary.

13. A method (100) in accordance with any of Claims 9 to 12, wherein collecting (118) and associating (120) are performed substantially simultaneously by collecting the projection data at at least one of predetermined angular positions that are indicated by the measured rotation parameter and a predetermined angular rate that is indicated by the measured rotation parameter.

14. A method (100) in accordance with any of Claims 9 to 13, wherein associating (120) the projection data with the measured rotation parameter comprises correcting the collected projection data using the measured rotation parameter at least one of during collection of the projection data and after collection of the projection data.

15. A method (100) in accordance with any of Claims 9 to 14, wherein associating (120) the projection data with the measured rotation parameter comprises time stamping at least one of the collected projection data and the measured rotation parameter.
